# FASCICULE DE BREVET EUROPEEN

(11) **EP 3 245 322 B1**
(45) Date de publication et mention de la délivrance du brevet: **27.11.2019**
(21) Numéro de dépôt: 16703580.7
(22) Date de dépôt: 15.01.2016
(51) Int. Cl.: D04H 1/49, B32B 25/10, B32B 27/12, D04H 1/558, D04H 1/70, B32B 5/02, A61F 13/49

(54) **NAPPE DE NON-TISSE RENFORCEE, ENSEMBLE COMPRENANT UNE TELLE NAPPE, ET PROCEDE DE TRAITEMENT D'UNE NAPPE DE NON-TISSE**
VERSTÄRKTER VLIESSTOFF, ANORDNUNG MIT SOLCH EINEM STOFF UND VERFAHREN ZUR BEHANDLUNG EINES VLIESSTOFFES
REINFORCED NON-WOVEN FABRIC, ASSEMBLY INCLUDING SUCH A FABRIC, AND METHOD FOR TREATING A NON-WOVEN FABRIC

(30) Priorité: 16.01.2015 FR 1550348
(43) Date de publication de la demande: 22.11.2017
(73) Titulaire: Aplix, 44850 Le Cellier (FR)
(72) Inventeur: MOINARD, Nathalie, 44980 Sainte Luce sur Loire (FR); MARCHE, Thierry, 44450 La Chapelle Basse Mer (FR)
(74) Mandataire: Urbain, Isabelle
(86) Numéro de dépôt international: PCT/FR2016/050082
(87) Numéro de publication internationale: WO 2016/113516

(56) Documents cités:
- EP-A1- 1 218 170
- EP-A1- 2 401 147
- WO-A1-01/00915
- WO-A1-03/008192

## Description

### DOMAINE TECHNIQUE

Le présent exposé concerne des nappes de non-tissé et des ensembles laminés comprenant de telles nappes.

Le présent exposé concerne, plus particulièrement, des nappes de non-tissé et des ensembles laminés pouvant être utilisés dans le domaine de l'hygiène, notamment pour la fabrication d'oreilles élastiques de couches culottes.

Dans le présent exposé, le terme non-tissé englobe tous les non-tissés tombant dans la définition communément admise par l'homme de l'art, typiquement des textiles constitués de fibres et/ou de filaments entremêlé(e)s et maintenu(e)s ensemble pour former une nappe.

### ETAT DE LA TECHNIQUE ANTERIEURE

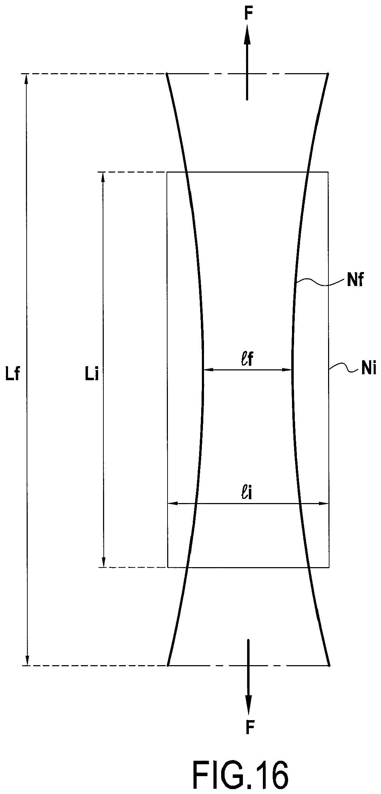

Sur les lignes de production d'ensembles laminés, et comme illustré sur la figure 16, une nappe de non-tissé est soumise, dans sa direction longitudinale (correspondant à la direction machine de la ligne, c'est-à-dire à la direction de défilement de la nappe sur la ligne), à une tension F.

Du fait de cette tension F, la nappe subit une réduction de sa largeur. Ce phénomène, connu sous le nom de « neckdown » ou « retreint », est illustré schématiquement par la figure 16. Dans son état initial, sans tension, la nappe (Nᵢ) présente une largeur lᵢ et une longueur Lᵢ. Une fois mise sous une tension longitudinale F, la longueur de la nappe (N_{f}) est augmentée (L_{f}) et sa largeur, réduite (l_{f}). Son épaisseur peut être augmentée elle aussi. Une solution pour pallier cette réduction de largeur des nappes consiste à utiliser, sur une ligne de production nécessitant une nappe de non-tissé de largeur utile lᵤ, une nappe de largeur effective lₐ supérieure à lᵤ, la différence entre la largeur utile lᵤ et la largeur effective lₐ correspondant à la diminution prévisionnelle de largeur de la nappe du fait du neckdown. Cette solution présente toutefois des inconvénients : à chaque variation de vitesse de la nappe lors d'un démarrage ou d'un arrêt de la ligne de production, la tension longitudinale appliquée sur la nappe est modifiée et la largeur de la nappe varie donc en conséquence. Pour que la largeur de la nappe en fin de ligne de production soit dans la plage de tolérance souhaitée, il est parfois nécessaire de découper les bords latéraux de la nappe. Mais même si de cette façon la largeur de la nappe peut être ajustée, ses performances mécaniques - notamment sa capacité d'élongation en direction transversale - peuvent varier localement selon l'ampleur du phénomène de neckdown, induisant également des variations locales de performances sur l'ensemble laminé incluant ladite nappe.

Une autre solution consiste à dérouler, sur les lignes de production, des nappes de non-tissé sans aucune tension, par exemple en les transportant sur un tapis. Pour former un ensemble laminé, ces nappes sont alors assemblées sans tension à un film élastique. Toutefois, lorsque l'ensemble laminé est déroulé par la suite, les nappes de non-tissé qui le constituent subissent le phénomène de neckdown, entraînant des déformations de l'ensemble laminé empêchant son bon guidage sur des lignes de production en aval.

### PRESENTATION DE L'INVENTION

L'un des objectifs de la présente invention est de fournir un procédé de traitement d'une nappe et une nappe ainsi traitée, permettant de remédier aux inconvénients de l'art antérieur énoncés ci-dessus.

Plus particulièrement, l'un des objectifs de l'invention est de permettre la maîtrise de la largeur d'une nappe de non-tissé lorsqu'une telle nappe est déroulée avec une tension sur une ligne de production.

Cet objectif est atteint avec une nappe de non-tissé s'étendant selon une direction longitudinale et une direction latérale orthogonale à la direction longitudinale, ladite nappe comprenant :
- au moins une zone de renforcement sur laquelle des fibres et/ou des filaments constituant la nappe sont liés selon un motif de renforcement comprenant une pluralité de formes géométriques, la zone de renforcement s'étendant sur toute la longueur de la nappe mesurée dans la direction longitudinale, et sur une largeur strictement inférieure à la largeur de la nappe mesurée dans la direction latérale, et
- au moins une zone non-renforcée,
de sorte que l'allongement de la zone renforcée sous l'effet d'une force donnée exercée dans la direction longitudinale est strictement inférieur à l'allongement de la zone non-renforcée, sous l'effet de la même force.

Par liaison de fibres et/ou filaments, on entend ici toute liaison augmentant leur cohésion. Plus particulièrement, une telle liaison peut comprendre une augmentation locale de la densité volumique de la nappe (notamment mais non exclusivement une augmentation locale de la densité de fibres et/ou de filaments), obtenue par toute liaison mécanique (par exemple par compression) et/ou thermique et/ou chimique des fibres et/ou filaments, ou encore par combinaison de plusieurs liaisons de ce type. Selon un exemple, la liaison peut être une soudure.

En conséquence, la rigidité de la nappe est plus importante sur la ou chaque zone de renforcement que sur les zones non-renforcées.

Lorsque la nappe est soumise à une tension longitudinale, son allongement dans la direction longitudinale est limité par rapport à une nappe de l'art antérieur non-renforcée subissant la même tension longitudinale, limitant également la déformation induite dans la direction latérale.

Dans le présent exposé, l'allongement d'une zone de la nappe est exprimé en pourcentage par rapport à sa longueur initiale.

Une nappe selon l'invention est par exemple constituée d'un non-tissé issu de la technologie Dry-laid (voie sèche), Wet-laid (voie humide), ou Spun-laid (voie fondue/extrudée) et consolidé par liaison mécanique, thermique, chimique et/ou adhésive.

Selon un exemple, la nappe est constituée d'un non-tissé de type cardé consolidé, notamment un non-tissé de type Spunlace, c'est-à-dire consolidé par hydro-liage.

Le motif de renforcement est généralement constitué par la répétition d'un motif élémentaire, dans la direction longitudinale de la nappe.

Selon l'invention, les formes géométriques constituant le motif de renforcement sont des éléments discrets, qui peuvent être par exemple des cercles, des croix, des losanges, des formes oblongues, etc.

On entend par « discrets » des éléments qui sont discontinus ou encore isolés, distincts les uns des autres. Ces éléments peuvent donc être délimités par un contour fermé.

On entend par « pluralité de formes géométriques », au moins deux éléments discrets dont les contours de chacun des au moins deux éléments discrets sont identiques, similaires ou différents entre eux.

Certaines formes peuvent être pleines. D'autres peuvent constituer une courbe fermée sur elle-même et présentant, sur toute son étendue, une bordure interne et une bordure externe.

Selon l'invention, les formes géométriques du motif de renforcement de chaque zone de renforcement sont agencées de sorte que toute droite s'étendant dans la direction latérale de la nappe coupe au moins l'une desdites formes. Le renforcement de la nappe est ainsi continu sur toute sa longueur. Des manifestations localisées du phénomène de neckdown sont ainsi évitées.

Selon un autre exemple, les formes géométriques du motif de renforcement de chaque zone de renforcement sont agencées de sorte que toute droite inclinée par rapport à la direction latérale d'un angle compris strictement entre 0 et 90°, plus particulièrement compris strictement entre 0° et 45°, encore plus particulièrement incliné d'un angle d'environ 24°, coupe au moins l'une desdites formes de ladite zone.

La zone de renforcement est généralement délimitée par une droite de délimitation à gauche DG et une droite de délimitation à droite DD, lesdites droites de délimitation, parallèles, s'étendant dans la direction longitudinale de la nappe. Le motif de soudure est entièrement contenu entre ces deux droites et chaque droite est tangente à au moins une forme du motif de renforcement.

Selon un exemple, le taux de liaison dans la zone de renforcement est supérieur à 10%, ou plus précisément supérieur à 15%, ou encore supérieur à 25%.

Selon un exemple, le taux de liaison dans la zone de renforcement est inférieur à 90%, ou plus particulièrement inférieur à 70%.

Dans le présent exposé, le taux de liaison sur la zone de renforcement est égal au pourcentage de surface dudit tronçon recouverte par les formes géométriques du motif de renforcement. Il est généralement mesuré sur un tronçon de la zone de renforcement de largeur égale à la largeur de ladite zone et de longueur égale à un nombre entier de motif élémentaire de renforcement.

Ce nombre entier est par exemple choisi de telle sorte que la longueur du tronçon soit supérieure à la largeur de la zone de renforcement.

Une première et une deuxième zone de renforcement adjacentes sont généralement séparées par une bande intermédiaire sans renforcement, s'étendant dans la direction longitudinale, et présentant préférentiellement une largeur au moins égale à 10% de la plus petite largeur parmi les largeurs des deux zones de renforcement.

Selon un exemple, la largeur de chaque zone de renforcement représente au plus 80% de la largeur de la nappe, de préférence au plus 60% de la largeur de la nappe.

Pour chaque zone de renforcement, on peut définir une portion utile, délimitée par une bordure de délimitation à gauche BG et une bordure de délimitation à droite BD.

La bordure de délimitation d'un côté de la portion utile est ainsi définie :
Elle comprend les points dits extrêmes du motif de renforcement, autrement dit les points qui se trouvent le plus à l'extérieur, dudit côté (dans la direction latérale), et ce pour chaque coordonnée prise le long d'un axe s'étendant dans la direction longitudinale de la nappe.

Elle comprend en outre, le cas échéant, des segments droits s'étendant dans la direction latérale et reliant ses portions constituées par les points extrêmes définis précédemment.

Chaque bordure de délimitation peut ainsi être une ligne droite, une ligne courbe, ou une combinaison d'un ou plusieurs segments droits et/ou courbes.

On peut mesurer le taux de liaison sur la portion utile de la zone de renforcement sur un tronçon de ladite portion utile s'étendant sur une longueur égale à la longueur du motif élémentaire. Il est égal au pourcentage de surface dudit tronçon recouverte par les formes géométriques du motif de renforcement.

Le taux de liaison sur la portion utile de la zone de renforcement est par exemple supérieur à 15%, ou plus précisément supérieur à 20%, ou encore supérieur à 35%.

Généralement, le taux de liaison sur la portion utile de la zone de renforcement est inférieur à 90%, plus particulièrement inférieur à 75%.

Selon un exemple, l'allongement de la zone renforcée sous l'effet d'une force de traction de 5 Newtons exercée dans la direction longitudinale est inférieur à l'allongement de la zone non-renforcée, sous l'effet de la même force.

L'allongement de la zone de renforcement sous l'effet de ladite force donnée exercée dans la direction longitudinale est par exemple inférieur d'au moins 5%, plus particulièrement inférieur d'au moins 15%, encore plus particulièrement inférieur d'au moins 50%, à l'allongement de la zone non-renforcée, sous l'effet de la même force. Par exemple, si la zone non-renforcée présente un allongement de 17% lorsqu'elle est soumise à une force de traction de 5 Newtons, alors l'allongement de la zone de renforcement est inférieur ou égal à 12%.

Selon un exemple, la nappe comprend en outre au moins une zone activée s'étendant sur une largeur inférieure à la largeur de la nappe, et sur laquelle le non-tissé est activé.

Une nappe de non-tissé présente généralement une capacité d'élongation faible au regard de celle d'un film élastique. Lorsqu'une telle nappe est destinée à être laminée sur un film élastique pour former un ensemble laminé, il est parfois nécessaire de lui appliquer au préalable, sur l'ensemble de sa surface ou localement, un traitement permettant de réduire la cohésion de sa structure, et d'assurer qu'une fois liée au film élastique, l'ensemble laminé puisse être étiré aisément (avec un moindre effort, par exemple 10N).

L'activation consiste à faire subir un étirement au non-tissé. Elle augmente, localement et de manière irréversible, la capacité du non-tissé à s'allonger. Typiquement, l'activation est réalisée dans la direction latérale de la nappe.

Un non-tissé activé présente généralement, vu en coupe transversale, sur sa zone activée, une forme de vagues lorsqu'il n'est soumis à aucune tension externe.

Le présent exposé concerne également un ensemble laminé comprenant au moins une première nappe telle que définie précédemment et au moins un film élastique relié à ladite première nappe.

Selon un exemple, l'ensemble laminé comprend au moins une deuxième nappe de non-tissé, le au moins un film élastique étant interposé entre la première et la deuxième nappe.

Selon un exemple, la première nappe comprend au moins une première zone de renforcement, et la deuxième nappe est une nappe du type défini précédemment, qui comprend au moins une deuxième zone de renforcement.

La première et la deuxième zone de renforcement peuvent par exemple se chevaucher sur au moins une zone de chevauchement de largeur prédéterminée.

Selon un exemple, les projections des motifs de renforcement respectifs de la première et de la deuxième zone de renforcement sont confondues sur au moins une zone de l'ensemble laminé.

On considère ici les projections des motifs de renforcement dans un plan orthogonal à l'épaisseur de l'ensemble laminé (autrement dit un plan orthogonal à la direction d'empilement des couches de l'ensemble laminé).

Selon un autre exemple, le motif de renforcement de la première zone de renforcement et le motif de renforcement de la deuxième zone de renforcement sont différents.

Selon un autre exemple, la projection, dans la direction Z, des motifs de renforcement respectifs de la première et de la deuxième zone de renforcement sont agencés de telle sorte que toute droite s'étendant dans la direction latérale de la nappe coupe la projection des formes géométriques des motifs de renforcement de la première et de la deuxième zone de renforcement. En particulier, la projection, dans la direction Z, des motifs de renforcement respectifs de la première et de la deuxième zone de renforcement sont agencés de telle sorte que toute droite inclinée par rapport à la direction latérale d'un angle compris strictement entre 0 et 90°, plus particulièrement compris strictement entre 0° et 45°, encore plus particulièrement incliné d'un angle d'environ 24°, coupe la projection des formes géométriques des motifs de renforcement de la première et de la deuxième zone de renforcement.

Chaque zone de renforcement de chaque nappe peut être décalée, dans la direction latérale, par rapport à une portion élastique de l'ensemble laminé.

Par portion élastique de l'ensemble laminé, on entend une portion de l'ensemble laminé comprenant le film élastique, et adapté à être étirée sous l'effet d'une force d'étirement exercée dans la direction latérale et à reprendre sensiblement sa forme et ses dimensions initiales après relâchement de ladite force d'étirement. Il s'agit par exemple d'une portion qui conserve une déformation résiduelle ou rémanence après élongation et relâchement (déformation résiduelle aussi appelée « permanent set » ou « SET ») inférieure à 20%, plus particulièrement inférieure à 15 %, dans certain cas inférieure à 5% de sa dimension initiale (avant élongation) pour un allongement de 100 % de sa dimension initiale, à température ambiante (23°C).

Selon un autre exemple, au moins une première zone de renforcement de la première nappe et au moins une deuxième zone de renforcement de la deuxième nappe se chevauchent sur au moins une zone de chevauchement de largeur prédéterminée, et l'une parmi la première zone de renforcement de la première nappe et la deuxième zone de renforcement de la deuxième nappe s'étend au-delà de ladite zone de chevauchement en direction d'une portion élastique de l'ensemble laminé, dans sa direction latérale.

Le présent exposé concerne également un procédé de traitement d'une nappe de non-tissé, ladite nappe s'étendant selon une direction longitudinale et une direction latérale orthogonale à la direction longitudinale, le procédé comprenant au moins une étape de renforcement au cours de laquelle, sur au moins une zone de renforcement de la nappe s'étendant sur toute la longueur de la nappe mesurée dans la direction longitudinale et sur une largeur strictement inférieure à la largeur de la nappe mesurée dans sa direction latérale, on lie des fibres et/ou filaments constituant la nappe selon un motif de renforcement comprenant une pluralité de formes géométriques, de sorte que l'allongement de la zone renforcée sous l'effet d'une force donnée exercée dans la direction longitudinale est inférieur à l'allongement d'une zone non-renforcée de la nappe, sous l'effet de la même force.

Durant l'étape de renforcement, on augmente la cohésion des fibres et/ou filaments de la nappe. Plus particulièrement, on augmente localement la densité volumique de la nappe (notamment mais non exclusivement on augmente localement la densité de fibres et/ou de filaments), par une liaison mécanique (par exemple en comprimant la nappe) et/ou thermique (en chauffant la nappe) et/ou chimique des fibres et/ou filaments, ou encore par combinaison de plusieurs liaisons de ce type. Les fibres et/ou filaments peuvent par exemple être soudé(e)s localement.

L'étape de renforcement peut par exemple être réalisée par calandrage à chaud.

Selon des variantes, elle peut aussi être réalisée par laser, ultrasons, embossage (calandrage à froid) ou encore une combinaison d'au moins deux de ces technologies.

Selon un exemple, le procédé comprend en outre une étape d'activation dans laquelle, sur au moins une zone activée de la nappe, on étire le non-tissé pour l'activer.

Selon un exemple, le procédé comprend en outre, préalablement à l'étape de renforcement, une étape d'ajustement de la largeur de la nappe.

Plusieurs exemples de réalisation sont décrits dans le présent exposé. Toutefois, sauf précision contraire, les caractéristiques décrites en liaison avec un exemple de réalisation quelconque peuvent être appliquées à un autre exemple de réalisation.

### BREVE DESCRIPTION DES DESSINS

L'invention sera bien comprise et ses avantages apparaîtront mieux, à la lecture de la description détaillée qui suit, de plusieurs modes de réalisation représentés à titre d'exemples non limitatifs. La description se réfère aux dessins annexés sur lesquels :
- La figure 1 illustre une nappe de non-tissé selon un mode de réalisation de la présente invention, en vue de dessus ;
- La figure 2 est une section transversale de la nappe de non-tissé de la figure 1, selon le plan II-II de la figure 1 ;
- La figure 3 représente la portion utile de la zone de renforcement, sur un tronçon de ladite zone correspondant à un motif élémentaire.
- Les figures 4A, 4B et 4C illustrent trois autres exemples de motif de renforcement ;
- La figure 5 est une section transversale d'un ensemble laminé selon un mode de réalisation de la présente invention ;
- La figure 6 est une section transversale d'un ensemble laminé selon un autre mode de réalisation de la présente invention ;
- La figure 7 est une section transversale, partielle, d'un ensemble laminé selon une variante de la figure 6 ;
- Les figures 8A et 8B illustrent respectivement la première zone de renforcement de la première nappe de non-tissé d'une variante de l'ensemble laminé de la figure 5 et la deuxième zone de renforcement de la deuxième nappe de non-tissé du même ensemble laminé ;
- La figure 8C illustre schématiquement la superposition de la première et de la deuxième zone de renforcement des figures 8A et 8B dans un plan orthogonal à la direction de l'épaisseur de l'ensemble laminé ;
- Les figures 9A et 9B illustrent respectivement la première zone de renforcement de la première nappe de non-tissé de l'ensemble laminé de la figure 6 et la deuxième zone de renforcement, identique, de la deuxième nappe de non-tissé du même ensemble laminé ;
- La figure 10 illustre schématiquement la superposition de la première et de la deuxième zone de renforcement des figures 9A et 9B dans un plan orthogonal à la direction de l'épaisseur de l'ensemble laminé ;
- La figure 11 illustre schématiquement la superposition de la première et de la deuxième zone de renforcement des figures 9A et 9B, selon un second exemple ;
- La figure 12 illustre schématiquement la superposition de la première et de la deuxième zone de renforcement des figures 9A et 9B, selon un troisième exemple ;
- La figure 13 illustre la superposition d'une première zone de renforcement d'une première nappe de non-tissé et d'une deuxième zone de renforcement d'une deuxième nappe de non-tissé d'un ensemble laminé selon un autre exemple de l'invention ;
- La figure 14 illustre schématiquement une installation de traitement d'une nappe de non-tissé, en vue de côté ;
- La figure 15 illustre, en vue de dessus, la nappe de non-tissé défilant à un instant t dans l'installation de traitement de la figure 14 ;
- La figure 16 décrite précédemment est un schéma de principe du phénomène de neckdown.

### DESCRIPTION DETAILLEE D'EXEMPLES DE REALISATION

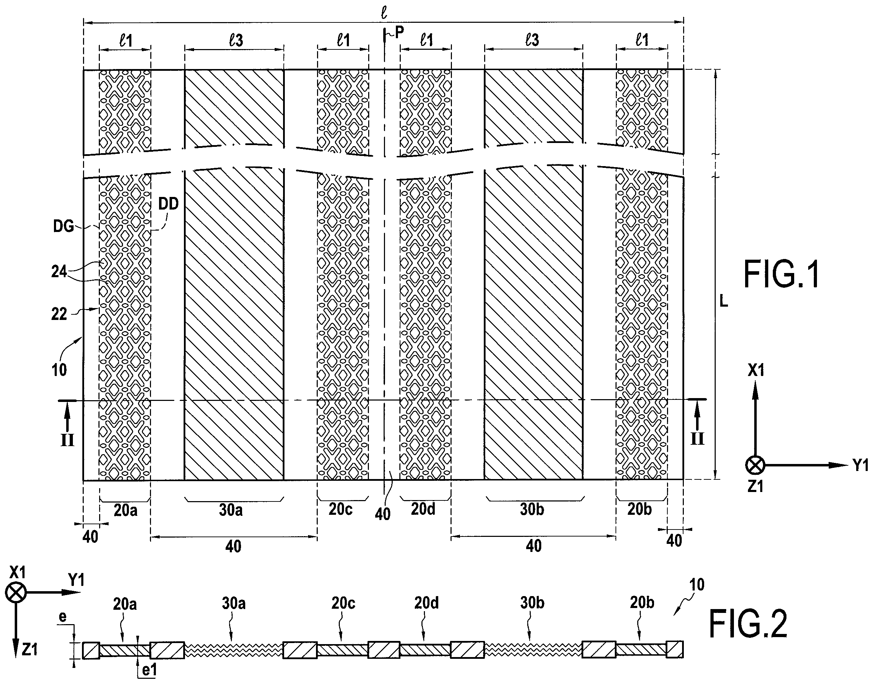

Sur les figures 1 et 2, on a représenté une nappe 10 de non-tissé selon un premier mode de réalisation de la présente invention.

On définit pour la nappe 10 une direction longitudinale X1 dans laquelle on mesure sa longueur L et une direction latérale Y1 orthogonale à X1, dans laquelle on mesure sa largeur I. Ces deux plus grandes dimensions de la nappe sont illustrées sur la vue de dessus de la figure 1.

La nappe 10 est par exemple réalisée en non-tissé de type Spunlace, constitué d'une pluralité de fibres consolidées par hydro-liage, et utilisé couramment dans le domaine de l'hygiène pour sa douceur et sa capacité naturelle de déformation.

Du fait de sa capacité élevée de déformation, le non-tissé Spunlace est particulièrement sujet au phénomène de neckdown décrit précédemment. Lorsqu'une tension lui est appliquée dans une direction donnée, il subit, dans la direction orthogonale, une déformation importante. Par conséquent, sur une ligne de production où elle est soumise à une forte tension longitudinale, une nappe en non-tissé Spunlace se rétrécit sensiblement dans le sens de la largeur.

Conformément à l'invention, la nappe 10 est localement renforcée, dans le but d'éviter le phénomène de neckdown.

Elle comporte ainsi, comme illustré sur la figure 1, des zones de renforcement 20a, 20b, 20c, 20d sur lesquelles des fibres constituant la nappe sont liées selon un motif de renforcement 22 comprenant une pluralité de formes géométriques 24, chaque zone de renforcement 20a, 20b, 20c, 20d s'étendant sur toute la longueur L de la nappe 10 et sur une largeur strictement inférieure à la largeur l de la nappe 10.

Les zones renforcées 20a, 20b, 20c, 20d sont bordées de zones non-renforcées 40.

Grâce à la liaison réalisée localement entre les fibres, la nappe 10 est rigidifiée dans les zones de renforcement 20a, 20b, 20c, 20d. Du fait de cette rigidification, l'allongement d'une zone renforcée de la nappe sous l'effet d'une force donnée exercée dans la direction longitudinale X1 est inférieur à l'allongement d'une zone non-renforcée, à force égale.

En conséquence, lorsque la nappe est soumise à une tension longitudinale, sa déformation induite dans la direction latérale est limitée par rapport à une nappe non-renforcée.

Pour mesurer l'allongement d'une zone de renforcement ou d'une zone non-renforcée, on peut par exemple utiliser la méthode suivante :
On conditionne la nappe de non-tissé dans une atmosphère normale, telle que définie dans la norme ASTDM 5170, à une température de 23°C ± 2°C et une humidité relative de 50% ± 5%.

On utilise comme appareillage un dynamomètre conforme à la norme EN 10002, notamment le Synergie 200H, disponible auprès de la société NTS Systems Corp, U.S.A., conjointement avec un logiciel d'utilisation TESTWORKS 4.04 B.

On prépare, à l'aide d'un cutter ou de ciseaux, un échantillon de 10mm de largeur dans le sens CD (direction transversale) de la nappe et de 150mm de longueur dans le sens MD (direction de la machine) de la nappe, dans une zone de renforcement ou une zone non-renforcée.

On positionne l'échantillon entre les mâchoires du dynamomètre.

Les paramètres sont sélectionnés comme suit :
Distance inter mâchoires : 100mm
Vitesse machine : 500 mm/mn
Nombre de cycles : 1
Pré-charge : 0.1N

On étire le produit dans le sens de sa largeur (correspondant à la direction longitudinale de la nappe) jusqu'à sa rupture, par déplacement vertical des mâchoires.

On obtient alors la courbe donnant la force d'étirement en fonction du pourcentage d'allongement. On peut ainsi définir le pourcentage d'allongement à 5N, qui correspond à l'allongement auquel est soumis le produit lors de son déroulage sur la ligne.

La valeur de 5N n'est pas limitative, et pourrait, dans d'autres procédés de mesure, être différente, par exemple égale à 10N.

On distingue, sur la figure 1, quatre zones de renforcement distinctes de la nappe 10 : deux zones de renforcement latérales 20a et 20b disposées en bordure latérale de la nappe et de largeur l1, et deux zones de renforcement centrales 20c, 20d de largeur l1 séparées l'une de l'autre par une zone non-renforcée 40.

La nappe présente ainsi un plan de symétrie P parallèle à sa direction longitudinale X1.

Le nombre, la largeur et la localisation des zones de renforcement d'une nappe selon l'invention peut cependant varier en fonction des besoins et de l'utilisation ultérieure prévue pour la nappe. Ainsi, une nappe peut comprendre une seule zone de renforcement ou un nombre de zones de renforcement différent de quatre. Par ailleurs, bien que ces configurations symétriques offrent des avantages, notamment celui de permettre la fabrication simultanée de deux oreilles élastiques identiques à partir d'un stratifié utilisant une telle nappe en réalisant une découpe selon le plan de symétrie P, la ou les zones de renforcement d'une nappe ne sont pas nécessairement en bordure latérale de cette nappe ou centrées sur cette nappe. Elles ne sont pas non plus réparties forcément selon une symétrie. Les largeurs des zones de renforcement sur une même nappe peuvent également être ajustées selon le cas.

Cependant, et comme illustré sur la figure 1, deux zones de renforcement adjacentes sont de préférence séparées par une zone non-renforcée 40 s'étendant dans la direction longitudinale et présentant une largeur au moins égale à 10% de la plus petite largeur parmi les largeurs des deux zones de renforcement.

Dans l'exemple, la nappe de non-tissé 10 comprend en outre, dans chaque zone non-renforcée 40 située entre deux zones de renforcement adjacentes 20a, 20b, 20c, une zone activée 30a, 30b sous forme de bande continue de largeur l3, sur laquelle les fibres du non-tissé ont été activées.

De préférence, la largeur l3 d'une zone activée est supérieure à la largeur l1 de la zone de renforcement. Plus particulièrement, la largeur l3 d'une zone activée est 1.5 fois plus large que la largeur l1 de la zone de renforcement.

Par souci de concision, une seule zone de renforcement 20a de la nappe 10 va être décrite plus en détail dans la suite. Tous les éléments décrits en liaison avec cette zone de renforcement 20a seront toutefois applicables aux autres zones de renforcement 20b, 20c de la nappe 10.

Comme illustré sur la figure 1, la zone de renforcement 20a se présente sous forme d'une bande délimitée par une droite de délimitation à gauche DG d'une part et une droite de délimitation à droite DD d'autre part, lesdites droites de délimitation, parallèles, s'étendant dans la direction longitudinale X1. Le motif de renforcement 22 est entièrement contenu entre ces deux droites DG, DD, et chaque droite est tangente à au moins une forme 24 du motif de renforcement 22.

De préférence, la largeur l1 de la zone de renforcement 20a - autrement dit la distance, mesurée dans la direction latérale Y1, entre les droites DG et DD - représente au plus 80% de la largeur l de la nappe 10, de préférence au plus 60% de la largeur l de la nappe 10.

Dans l'exemple, au droit du motif de renforcement 22, les fibres de la nappe 10 sont comprimées et soudées les unes aux autres, pouvant aller jusqu'à la fusion complète de la matière les constituant, la disparition des fibres en tant que telles et leur remplacement par une zone de film, augmentant localement ainsi la densité volumique de la nappe. Un tel renforcement est par exemple obtenu par calandrage à chaud, comme il sera décrit plus en détail dans la suite.

L'épaisseur e1 de la nappe 10 au niveau de la zone de renforcement 20a est inférieure à l'épaisseur e de la nappe dans les zones non-renforcées 40, comme illustré sur la figure 2.

Le motif de renforcement 22, contenu dans la zone 20a, est typiquement constitué par la répétition régulière, dans la direction longitudinale X1, d'un motif élémentaire 26 de longueur lm.

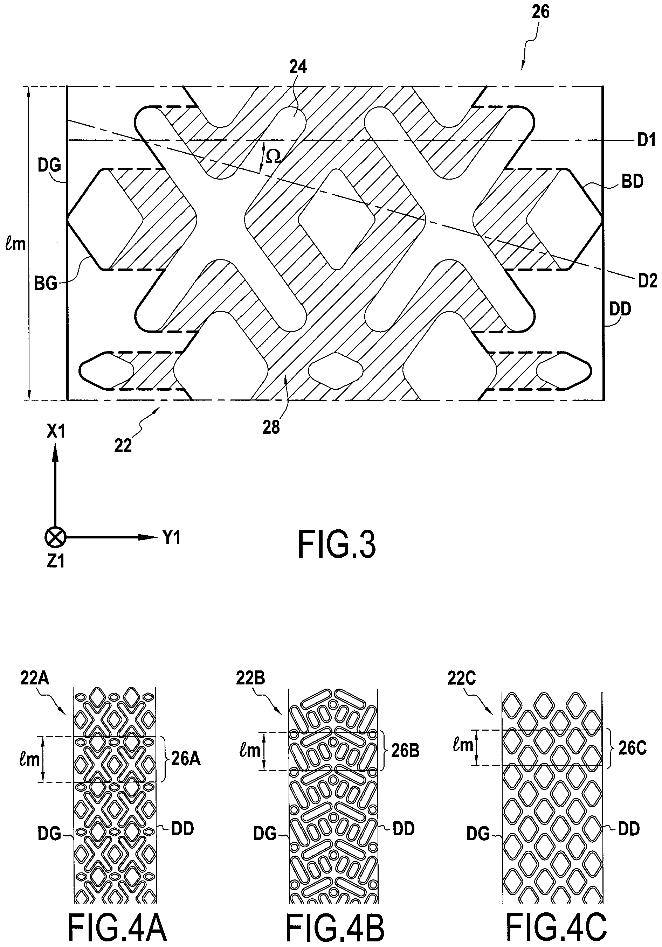

Le motif élémentaire de renforcement 26 de la zone 20a est illustré plus en détail sur la figure 3.

Dans l'exemple, il est constitué d'une combinaison de formes géométriques discrètes, pleines, ici des croix et des losanges.

Le taux de liaison dans la zone de renforcement 20a, autrement dit le taux de liaison sur un tronçon de la zone de renforcement correspondant au motif élémentaire 26, est de préférence supérieur à 10% et inférieur à 90%, ou plus précisément compris entre 25% à 70%.

On définit également une portion utile 28 de la zone de renforcement 20a, délimitée par une bordure de délimitation à gauche BG et une bordure de délimitation à droite BD. Dans l'exemple de la figure 1, les bordures de délimitation BG, BD s'étendent généralement dans la direction longitudinale X1.

Les bordures de délimitation à gauche et à droite BG, BD de la portion utile 28 sont illustrées sur la figure 3.

La bordure à gauche BG comprend les points extrêmes du motif de renforcement 22 qui se trouvent le plus à gauche (dans la direction latérale Y1), et ce pour chaque coordonnée prise le long d'un axe s'étendant dans la direction longitudinale X1. Ces points extrêmes sont représentés en trait gras continus, à gauche, sur la figure 3. Elle comprend en outre des segments droits (représentés en traits gras discontinus, à gauche, sur la figure 3) s'étendant dans la direction latérale Y1 et reliant les portions de bordure constituées par les points extrêmes, à gauche, définis ci-dessus.

La bordure à droite BD comprend les points extrêmes du motif de renforcement 22 qui se trouvent le plus à droite (dans la direction latérale Y1), et ce pour chaque coordonnée prise le long d'un axe s'étendant dans la direction longitudinale X1. Ces points extrêmes sont représentés en trait gras continus, à droite, sur la figure 3. Elle comprend en outre des segments droits (représentés en traits gras discontinus, à droite, sur la figure 3) s'étendant dans la direction latérale Y1 et reliant les portions de bordure constituées par les points extrêmes, à droite, définis ci-dessus.

Le taux de liaison sur la portion utile 28 de la zone de renforcement 20a, mesuré sur un tronçon de longueur Lm, (ici le motif élémentaire) est de préférence supérieur à 15%, plus particulièrement supérieur à 20%, et inférieur à 90%.

Comme il est bien connu de l'homme de l'art, le maintien des fibres et/ou filaments d'un non-tissé résulte d'une consolidation réalisée sur toute l'étendue de fibres et/ou filaments entremêlé(e)s lors de la fabrication du non-tissé, consolidation qui peut être thermique (calandrage, ultrasons, etc.), mécanique (hydro-liage, aiguilletage, etc.), chimique ou adhésive.

La consolidation assure une certaine cohésion des fibres et/ou filaments, permettant leur manipulation et leur transport, notamment leur enroulement sous forme de bobine et leur déroulement. Elle peut dans certains cas être réalisée en une pluralité de points répartis de manière sensiblement homogène sur toute la largeur de la nappe de non-tissé. Les non-tissés cardés calandrés, par exemple, présentent typiquement des points de consolidation thermique.

Les points de consolidation forment un motif différent du motif de renforcement. Par souci de simplification, ces points de consolidation du non-tissé ne sont pas représentés sur les figures.

Il est entendu que les points de consolidation sont clairement à distinguer du motif de renforcement décrit précédemment. La consolidation permet de donner une cohésion initiale à la nappe, sur toute sa surface, pour former un non-tissé, tandis que le renforcement apporté par la présente invention permet d'augmenter localement la cohésion initiale résultant de cette consolidation. Les points de consolidation du non-tissé ne sont donc pas pris en compte dans le calcul des taux de liaison du renforcement mentionnés précédemment.

Selon un exemple, les formes géométriques 24 du motif de renforcement 22 sont agencées de sorte que toute droite s'étendant dans la direction latérale Y1 de la nappe 10, telle que D1 sur la figure 3, coupe au moins l'une desdites formes 24. Le renforcement de la nappe 10 est ainsi continu sur toute sa longueur. Des manifestations localisées du phénomène de neckdown sont ainsi évitées.

Encore plus préférentiellement, les formes géométriques 24 du motif de renforcement 22 sont agencées de sorte que toute droite inclinée par rapport à la direction latérale Y1 d'un angle Ω compris strictement entre 0 et 90°, telle que D2 sur la figure 3, coupe au moins l'une desdites formes 24. En particulier, l'angle Ω est compris strictement entre 0 et 45°.

Le motif de renforcement 22 décrit précédemment n'est toutefois pas limitatif, et les formes géométriques 24, ainsi que leur agencement, peuvent varier.

En particulier, les formes géométriques 24 constituant le motif de renforcement 22 peuvent ne pas être pleines. Ainsi, selon une variante, chaque forme 24 peut être constituée par une courbe fermée sur elle-même et présentant, sur toute son étendue, une bordure interne et une bordure externe.

C'est le cas du motif de renforcement 22A illustré sur la figure 4A : chaque croix ou losange 24A est ici constitué(e) par une courbe formant un contour fermé.

Le choix de ce type de motif permet de limiter l'énergie nécessaire à sa création tout en conservant des performances satisfaisantes. Par ailleurs, la surface reste douce au toucher.

Les figures 4B et 4C illustrent deux autres exemples de motifs 22B, 22C, comprenant respectivement une combinaison de cercles et de formes oblongues (figure 4B), et une succession de losanges identiques (figure 4C).

Selon un autre exemple, le motif de renforcement 22 peut aussi comprendre des bordures de délimitation BG, BD s'étendant selon des directions sensiblement inclinées par rapport à la direction longitudinale X1, par exemple déviant par rapport à cette direction alternativement vers la gauche puis vers la droite, à la manière d'un zigzag ou d'une sinusoïde. Une telle disposition peut être obtenue à l'aide de rouleaux de calandrage pivotant de la gauche vers la droite ou de la droite vers la gauche, pendant la réalisation d'un motif élémentaire de renforcement.

Une nappe renforcée 10, du type décrit ci-dessus, peut être utilisée dans la fabrication d'ensembles laminés, et notamment de trilaminés comprenant deux nappes de non-tissé, et au moins un film élastique interposé entre lesdites nappes.

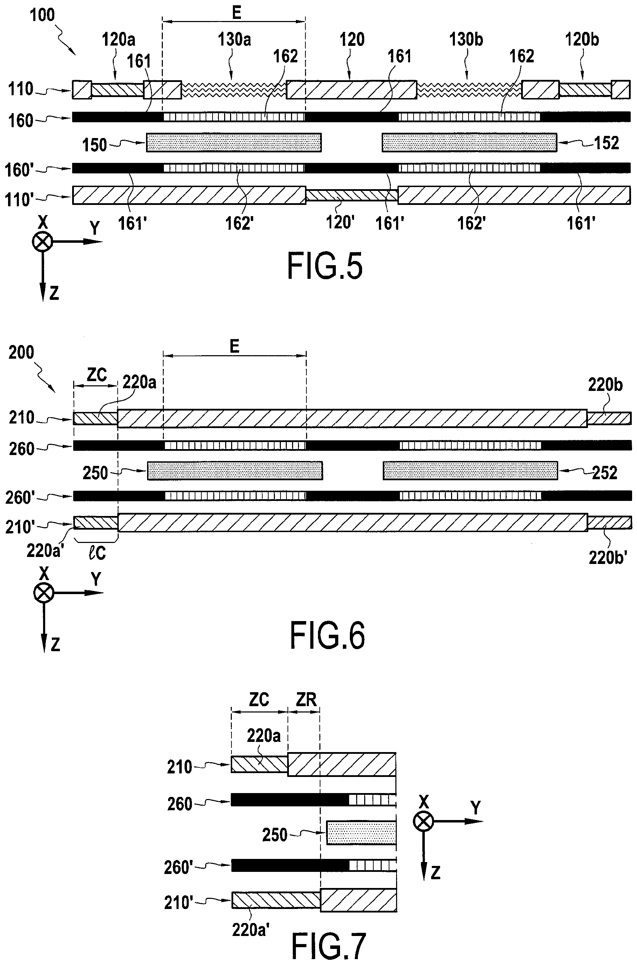

Un premier mode de réalisation d'un tel ensemble laminé 100 est illustré sur la figure 5. De façon évidente, les directions longitudinale X et latérale Y d'un ensemble laminé doivent être considérées, dans la suite, comme parallèles respectivement aux directions longitudinales et latérales des nappes formant cet ensemble laminé. La direction Z de l'épaisseur de l'ensemble laminé est définie comme la direction d'empilement des différentes couches de cet ensemble.

L'ensemble laminé 100 est formé par superposition, dans la direction Z :
- d'une première nappe de non-tissé 110 renforcée, munie de deux zones de renforcement 120a, 120b disposées le long des bordures latérales de la nappe 110, ainsi que deux zones activées 130a, 130b disposées entre lesdites zones de renforcement 120a, 120b,
- de deux films élastiques 150, 152 disposés sensiblement au droit de la première et de la deuxième zone activée 130a, 130b de la première nappe 110, et
- d'une deuxième nappe de non-tissé renforcée 110', munie d'une unique zone de renforcement 120', centrée sur ladite nappe 110'.

Les nappes de non-tissé 110, 110' de l'ensemble laminé et les films élastiques 150, 152 interposés entre lesdites nappes, sont reliés par de la colle 160, 160'.

Les zones activées 130a, 130b de la première nappe 110 lui confèrent localement une certaine capacité d'élongation.

La deuxième nappe, réalisée dans un non-tissé par nature plus élastique que celui constituant la première nappe 110, n'est pas activé.

Selon une variante de réalisation cependant, les deux nappes pourraient aussi être réalisées dans un non-tissé de même nature.

Dans l'exemple, la colle 160, 160' est appliquée en bandes pleines 161, 161' entre les films élastiques et sur les bordures latérales de ceux-ci, ce par quoi les films élastiques sont fixés fermement aux nappes 110, 110', et en lignes étroites ou filets 162, 162' au droit des zones activées 130a, 130b de la première nappe 110. On pourrait, selon une variante, encoller le film élastique sur toute sa largeur de manière continue, en choisissant éventuellement une épaisseur de colle plus faible sur certaines zones (notamment au centre).

Dans l'exemple, les films élastiques 150, 152 peuvent être étirés entre les filets de colle 162, 162', assurant l'élasticité de l'ensemble laminé 100.

Les zones E situées au droit des zones centrales des films élastiques 150, 152 recouvertes de filets de colles 162, 162' constituent donc des portions dites élastiques de l'ensemble laminé 100.

Dans un autre mode de réalisation, on peut envisager que le film élastique soit appliqué directement sur les nappes de non tissé, et ainsi obtenir un stratifié dépourvu de colle (agent de fixation), par exemple en rapportant les nappes sur le film élastique en sortie d'extrudeuse.

Par exemple, comme illustré sur la figure 5, chaque zone de renforcement 120a, 120b, 120' de chaque nappe 110, 110' est décalée, dans la direction latérale Y, par rapport à ces portions élastiques E.

On remarque en outre, dans l'exemple de la figure 5, que les zones de renforcement 120a, 120b de la première nappe 110 sont décalées par rapport à celle 120' de la deuxième nappe 110', dans la direction latérale Y.

Selon d'autres exemples de réalisation, une première zone de renforcement de la première nappe et une deuxième zone de renforcement de la deuxième nappe peuvent se chevaucher, autrement dit être positionnées en regard l'une de l'autre, dans la direction Z.

Dans ce cas, les motifs de renforcement des deux zones superposées peuvent être identiques ou différents.

Par ailleurs, les deux zones de renforcement peuvent ou non présenter la même largeur, et les deux zones peuvent se chevaucher sur toute leur largeur ou sur une partie seulement de leur largeur.

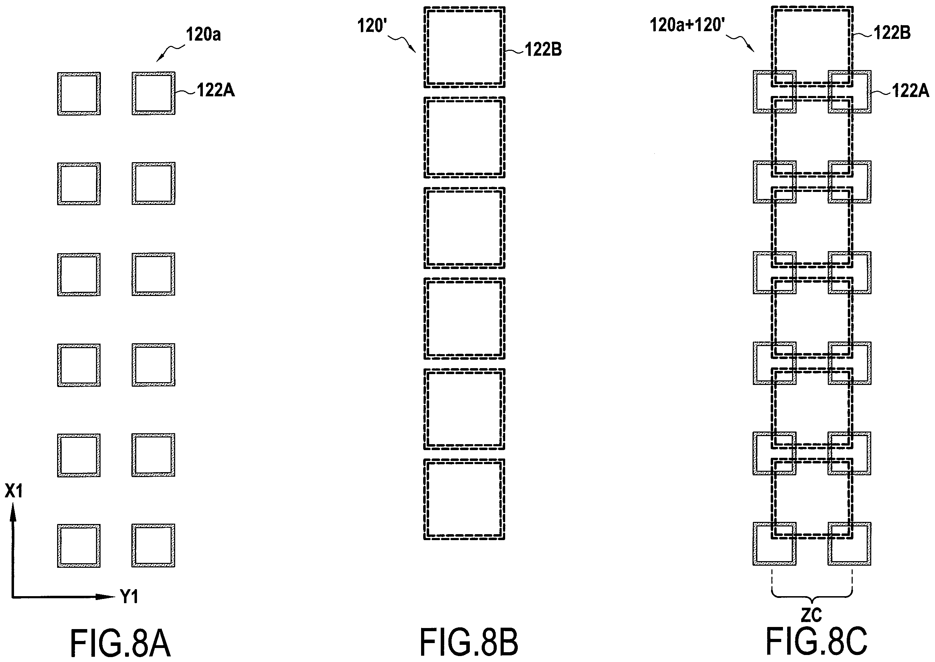

Ainsi, par exemple, la figure 8A illustre un motif de renforcement 122A de la première zone de renforcement 120a de la première nappe 110 et la figure 8B illustre un motif de renforcement 122B de la deuxième zone de renforcement 120' de la deuxième nappe 110', les deux zones de renforcement 120a, 120' présentant des motifs géométriques différents 122A et 122B.

Dans ce mode de réalisation, les première et deuxième zones de renforcement 120a, 120' s'étendent sur toute la longueur de la nappe mesurée dans la direction longitudinale et sur une largeur strictement inférieure à la largeur de la nappe mesurée dans la direction latérale.

La figure 8C illustre, quant à elle, les première et deuxième zones de renforcement 120a, 120' qui ne présentent pas la même largeur et qui se chevauchent sur toute la largeur de la zone de renforcement 120' et sur une partie seulement de la largeur de la zone de renforcement 120a. Les première et deuxième zones de renforcement 120a, 120' se recouvrent dans une zone de chevauchement centrale ZC.

On constate que, dans cet exemple, la projection, dans la direction Z, des motifs de renforcement respectifs 122A, 122B de la première et de la deuxième zone de renforcement 120a, 120' sont agencés de telle sorte que toute droite s'étendant dans la direction latérale Y1 de la nappe coupe la projection des formes géométriques des motifs de renforcement de la première et de la deuxième zone de renforcement. En particulier, la projection, dans la direction Z, des motifs de renforcement 122A, 122B respectifs de la première et de la deuxième zone de renforcement 120a, 120' sont agencés de telle sorte que toute droite inclinée par rapport à la direction latérale d'un angle compris strictement entre 0 et 90°, plus particulièrement compris strictement entre 0° et 45°, encore plus particulièrement incliné d'un angle d'environ 24°, coupe la projection des formes géométriques des motifs de renforcement de la première et de la deuxième zone de renforcement.

Selon une disposition particulière, les zones de renforcement peuvent présenter des motifs de renforcement identiques ou partiellement identiques, et les projections, dans la direction de l'empilement, des motifs de renforcement respectifs de la première et de la deuxième zone de renforcement peuvent être confondues sur au moins une largeur prédéterminée de l'ensemble laminé.

La figure 6 illustre un ensemble laminé 200 selon un autre mode de réalisation de l'invention, comprenant :
- une première nappe de non-tissé 210 renforcée, munie de deux zones de renforcement 220a, 220b disposées le long des bordures latérales de la nappe 210,
- une deuxième nappe de non-tissé renforcée 210', munie de deux zones de renforcement 220b, 220b' disposées le long des bordures latérales de la nappe 210',
- deux films élastiques 250, 252 interposés entre les deux nappes 210, 210',
- de la colle 260, 260' reliant entre eux les films élastiques 250, 252 et les nappes de non-tissé 210, 210'.

La structure générale de l'ensemble laminé, notamment en ce qui concerne la disposition des lignes et bandes de colle, est sensiblement identique à celle de la figure 5 et n'est donc pas décrite à nouveau.

Une première zone de renforcement 220a appartenant à la première nappe chevauche ici une deuxième zone de renforcement 220a' appartenant à la deuxième nappe 210', sur une zone de chevauchement notée ZC sur la figure 6.

Le côté opposé de l'ensemble laminé étant agencé symétriquement, il n'est pas décrit plus en détail dans la suite.

Dans le cas particulier représenté, les zones de renforcement 220a et 220a' présentent une même largeur, et se chevauchent sur toute cette largeur notée lc (largeur de la zone de chevauchement).

Selon une disposition particulière, les motifs de renforcement de la première et de la deuxième zone de renforcement sont identiques.

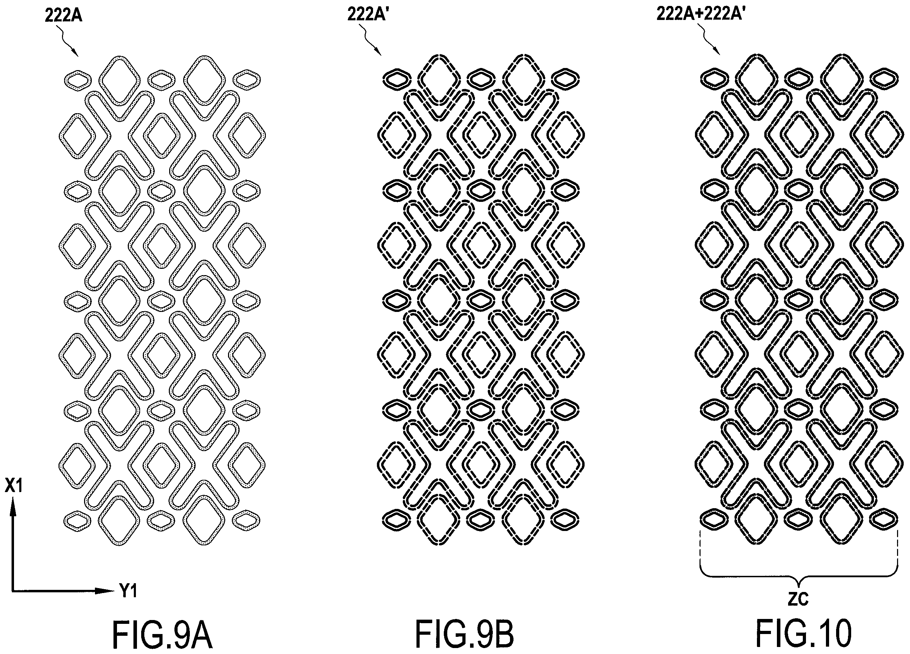

Ainsi, par exemple, la figure 9A illustre le motif de renforcement 222A de la première zone de renforcement 220a de la première nappe 210, et la figure 9B illustre le motif de renforcement 222A' de la deuxième zone de renforcement 220a' de la deuxième nappe 210'.

Selon un exemple, les projections, dans un plan orthogonal à la direction Z de l'épaisseur de l'ensemble laminé 200, des motifs de renforcement 222A, 222A' de la première et de la deuxième zone de renforcement 220a, 220a' sont confondues sur la zone de chevauchement ZC, comme illustré par la figure 10.

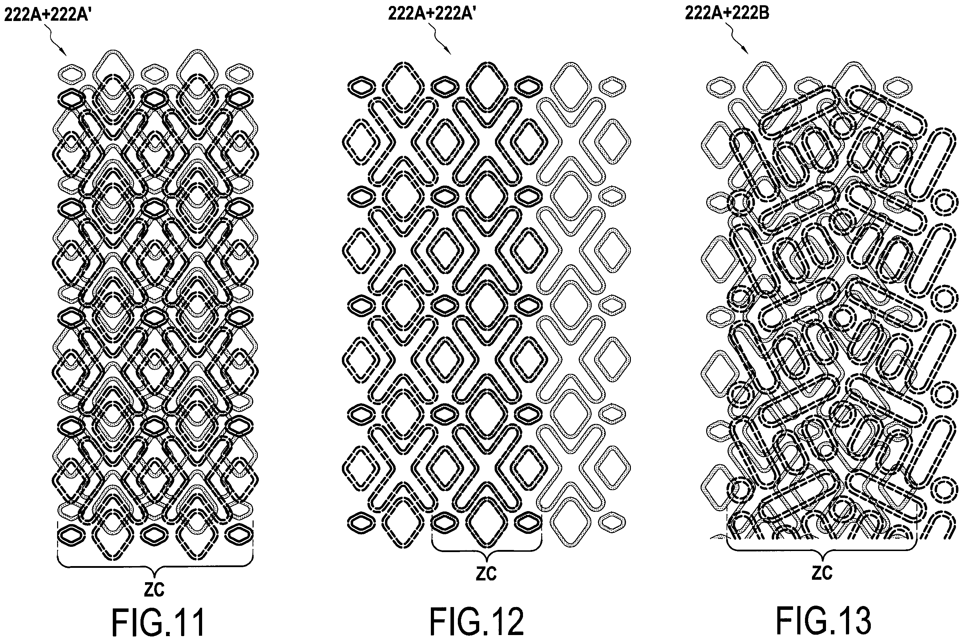

Selon un autre exemple illustré sur la figure 11, les motifs de renforcement respectifs 222A, 222A' de la première et de la deuxième zone de renforcement 220a, 220a' peuvent aussi être décalés dans la direction longitudinale X de l'ensemble laminé, de sorte que leurs projections ne sont plus confondues.

Selon encore un autre exemple illustré sur la figure 12, les deux zones de renforcement 220a, 220a' peuvent être décalées latéralement l'une par rapport à l'autre, les projections de leur motif de renforcement 222A, 222A' restant confondues sur une zone de chevauchement ZC de largeur lc inférieure à la largeur de leur motif commun.

La figure 13 illustre, quant à elle, deux zones de renforcement présentant des motifs différents 222A, 222B, mais décalées l'une par rapport à l'autre de façon à se recouvrir dans une zone de chevauchement centrale ZC.

La figure 7 illustre une variante du mode de réalisation de la figure 6, présentant une disposition particulière des zones de renforcement des nappes d'un non-tissé.

Comme illustré sur cette figure 7, la deuxième zone de renforcement 220a' de la deuxième nappe de non-tissé 210' s'étend au-delà de la zone de chevauchement ZC, dans la direction latérale Y1, en direction d'une portion élastique E de l'ensemble laminé.

Dans l'exemple, on note ZR la portion de la deuxième zone de renforcement 220a' dépassant de la zone de chevauchement. Cette portion fait face à une zone non-renforcée de la première nappe 210. Elle est disposée, dans la direction latérale Y1, entre la zone de chevauchement ZC et le film élastique 250.

Il a été constaté que de telles dispositions permettent de limiter les concentrations de contraintes dans l'ensemble laminé, et d'augmenter sa résistance à la rupture.

A noter que l'exemple de la figure 7 n'exclut pas qu'une zone de renforcement dépasse également de la zone de chevauchement ZC du côté de ladite zone opposé au film élastique 250, dans la direction latérale Y1.

Par ailleurs, la zone de renforcement s'étendant au-delà de la zone de chevauchement ZC en direction d'une portion élastique E de l'ensemble laminé pourrait tout aussi bien être la première zone de renforcement 220a de la première nappe 210.

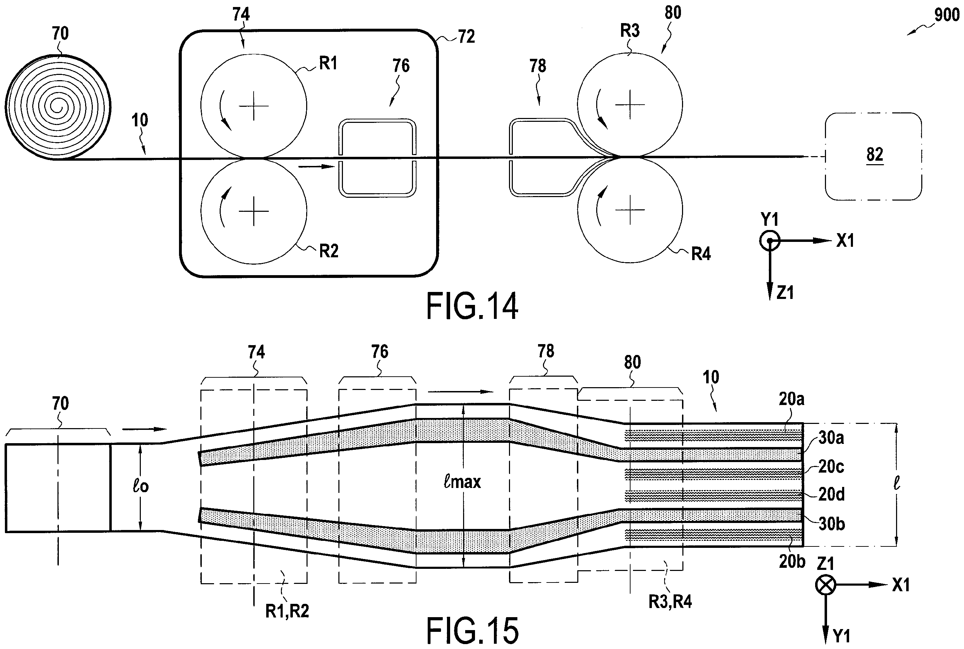

Sur la figure 14, on a représenté une installation de traitement d'une nappe de non-tissé 900, pouvant notamment être utilisée en amont d'une ligne de production d'un ensemble laminé pour la réalisation d'une nappe renforcée 10 du type décrit en liaison avec les figures 1 et 2.

L'installation 900 comprend, de l'amont vers l'aval dans la direction de défilement de la nappe à traiter (de la gauche vers la droite, sur la figure):
- un poste de déroulement de la nappe de non-tissé 70, initialement conditionnée sous forme de bobine,
- un module d'élargissement de la nappe 72, comprenant
   ∘ un module d'activation 74 destiné à activer des zones localisées de la nappe, formant les zones 30a, 30b dites activées de la nappe 10, et
   ∘ un module d'étirement 76 pour étirer la nappe 10 dans sa direction latérale,
- un module de gestion de la largeur de la nappe 78, et
- un module de renforcement de la nappe 80.

Selon un exemple, le procédé de traitement de la nappe comprend les étapes suivantes :
La nappe 10, une fois déroulée, est activée localement par le module d'activation 74. Dans l'exemple, ce module 74 comprend deux rouleaux d'activation R1, R2 munis chacun d'un empilement de disques parallèles. Les disques de chaque rouleau engrenant avec les disques du rouleau adjacent, les zones de la nappe 10 passant entre les disques des rouleaux R1, R2 sont étirées dans la direction latérale Y1, formant les zones activées 30a, 30b. A ces endroits, les fibres de la nappe 10 sont cassées.

La nappe 10 est ensuite déformée plus globalement par le module d'étirement 76 qui comprend généralement une pluralité de rouleaux (non représentés) ayant pour but d'étirer latéralement la nappe. Elle y est étirée jusqu'à prendre une largeur lmax supérieure à la largeur utile l souhaitée pour l'ensemble laminé fabriqué en aval sur la ligne de production 82.

Dans le module de gestion de la largeur 78, qui comprend des moyens de mesure de la largeur de la nappe et des moyens d'ajustement, par exemple par étirage, de cette largeur à une valeur souhaitée, la largeur de la nappe 10 est ajustée à la largeur utile l qu'elle devra conserver sur toute la ligne de production 82.

Elle est ensuite renforcée, immédiatement en aval du module de gestion de la largeur 78, afin d'éviter toute variation de sa largeur.

Le renforcement est réalisé par exemple par calandrage à chaud.

Dans ce cas, le module de renforcement 80 comprend deux rouleaux de calandrage R3, R4 chauffés formant rouleaux de renforcement, dont l'un au moins comporte, sur sa surface externe, des bagues (ici quatre) munies de nervures reproduisant un motif élémentaire de renforcement. Lors du passage de la nappe 10 à travers les rouleaux de renforcement, dans sa direction longitudinale, les nervures appuient sur les fibres qui, sous l'effet de la chaleur, se déforment et se soudent entre elles, formant les zones de renforcement 20a, 20b, 20c, 20d.

Selon des variantes de mise en œuvre, le renforcement pourrait par exemple être réalisé par laser, ultrasons, embossage ou encore une combinaison d'au moins deux de ces technologies.

## Revendications

1. Nappe (10, 110, 110', 210, 210') de non-tissé s'étendant selon une direction longitudinale (X1) et une direction latérale (Y1) orthogonale à la direction longitudinale (X1), ladite nappe comprenant :
- au moins une zone de renforcement (20a, 20b, 20c) sur laquelle des fibres et/ou des filaments constituant la nappe sont liés selon un motif de renforcement (22) comprenant une pluralité de formes géométriques (24), la zone de renforcement s'étendant sur toute la longueur (L) de la nappe mesurée dans la direction longitudinale (X1), et sur une largeur (l1, l2) strictement inférieure à la largeur (l) de la nappe mesurée dans la direction latérale (Y1), et
- au moins une zone non-renforcée (40),
l'allongement de la zone renforcée (20a, 20b, 20c, 20d) sous l'effet d'une force donnée exercée dans la direction longitudinale (X1) étant inférieur à l'allongement de la zone non-renforcée (40), sous l'effet de la même force,
les formes géométriques (24) du motif de renforcement (22) étant des éléments discrets, et
les formes géométriques (24) du motif de renforcement (22) de chaque zone de renforcement (20a, 20b, 20c, 20d) étant agencées de sorte que toute droite (D1) s'étendant dans la direction latérale (Y1) de la nappe coupe au moins l'une desdites formes (24).

2. Nappe (10, 110, 110', 210, 210') selon la revendication 1, dans laquelle le taux de liaison de la zone de renforcement (20a, 20b, 20c, 20d) est supérieur à 10%, plus préférentiellement supérieur à 15%.

3. Nappe (10, 110, 110', 210, 210') selon la revendication 1 ou 2, dans laquelle le taux de liaison dans une portion utile de la zone de renforcement (20a, 20b, 20c, 20d) est supérieur à 15%, plus préférentiellement supérieur à 20%, et/ou dans laquelle le taux de liaison dans une portion utile de la zone de renforcement est inférieur à 90%.

4. Nappe (10, 110, 110', 210, 210') selon l'une quelconque des revendications 1 à 3, dans laquelle la largeur (l1, l2) de chaque zone de renforcement (20a, 20b, 20c, 20d) représente au plus 80% de la largeur (l) de la nappe, de préférence au plus 60% de la largeur (l) de la nappe.

5. Nappe (10, 110, 110', 210, 210') selon l'une quelconque des revendications 1 à 4, dans laquelle l'allongement à 5N de la zone de renforcement (20a, 20b, 20c, 20d) est inférieur à l'allongement à 5N de la zone non-renforcée (40), de préférence inférieur d'au moins 5% à l'allongement de la zone non-renforcée (40).

6. Nappe (10, 110, 110', 210, 210') selon l'une quelconque des revendications 1 à 5, consistant en un non-tissé de type cardé consolidé, notamment un non-tissé de type Spunlace.

7. Ensemble laminé (100, 200) comprenant au moins une première nappe (110, 210) selon l'une quelconque des revendications précédentes et au moins un film élastique (150, 152, 250, 252) relié à ladite première nappe.

8. Ensemble laminé (100, 200) selon la revendication 7, comprenant au moins une deuxième nappe (110', 210') de non-tissé, le au moins un film élastique (150, 152, 250, 252) étant interposé entre la première et la deuxième nappe (110, 110', 210, 210').

9. Ensemble laminé (100, 200) selon la revendication 8, dans lequel la première nappe (110, 210) comprend au moins une première zone de renforcement (120a, 120b, 220a, 220b), et la deuxième nappe (110', 210') est une nappe selon l'une quelconque des revendications 1 à 9, qui comprend au moins une deuxième zone de renforcement (120', 220a', 220b').

10. Ensemble laminé (100, 200) selon la revendication 9, dans lequel la première et la deuxième zone de renforcement (120a, 120', 220a, 220a') se chevauchent sur au moins une zone de chevauchement (ZC) de largeur prédéterminée (lc).

11. Ensemble laminé (200) selon la revendication 10, dans lequel les projections des motifs de renforcement respectifs (222A, 222A') de la première et de la deuxième zone de renforcement (220a, 220a') sont confondues sur au moins une zone de l'ensemble laminé.

12. Ensemble laminé (100, 200) selon la revendication 10, dans lequel la projection, dans la direction Z, des motifs de renforcement (122A, 122B) respectifs de la première et de la deuxième zone de renforcement sont agencés de telle sorte que toute droite s'étendant dans la direction latérale de la nappe coupe la projection des formes géométriques des motifs de renforcement de la première et de la deuxième zone de renforcement.

13. Ensemble laminé (100, 200) selon l'une quelconque des revendications 7 à 12, dans lequel chaque zone de renforcement (120a, 120b, 220a, 220b ; 120', 220a', 220b') de chaque nappe (110, 110', 210, 210') est décalée, dans la direction latérale (Y1), par rapport à une portion élastique (E) de l'ensemble laminé (100, 200).

14. Procédé de traitement d'une nappe (10) de non-tissé, ladite nappe s'étendant selon une direction longitudinale (X1) et une direction latérale (Y1) orthogonale à la direction longitudinale (X1), le procédé comprenant au moins une étape de renforcement au cours de laquelle, sur au moins une zone de renforcement (20) de la nappe (10) s'étendant sur toute la longueur de la nappe mesurée dans la direction longitudinale et sur une largeur (l1) strictement inférieure à la largeur (l) de la nappe (10) mesurée dans sa direction latérale, on lie des fibres et/ou filaments constituant la nappe (10) selon un motif de renforcement (22) comprenant une pluralité de formes géométriques (24), les formes géométriques (24) du motif de renforcement (22) étant des éléments discrets, et les formes géométriques (24) du motif de renforcement (22) de chaque zone de renforcement (20a, 20b, 20c, 20d) étant agencées de sorte que toute droite (D1) s'étendant dans la direction latérale (Y1) de la nappe coupe au moins l'une desdites formes (24), de sorte que l'allongement de la zone renforcée sous l'effet d'une force donnée exercée dans la direction longitudinale est inférieur à l'allongement d'une zone non-renforcée de la nappe, sous l'effet de la même force, l'étape de renforcement étant de préférence réalisée par calandrage à chaud.

15. Procédé de traitement selon la revendication 14, comprenant en outre, préalablement à l'étape de renforcement, une étape d'ajustement de la largeur (l) de la nappe.

## Patentansprüche

1. Vliesstoff (10, 110, 110', 210, 210'), der sich entlang einer Längsrichtung (X1) und einer seitlichen Richtung (Y1) erstreckt, die orthogonal zur Längsrichtung (X1) ist, wobei der Stoff umfasst:
- mindestens einen Verstärkungsbereich (20a, 20b, 20c), auf dem Fasern und/oder Filamente, die den Stoff bilden, gemäß einem Verstärkungsmotiv (22) verbunden sind, das mehrere geometrische Formen (24) umfasst, wobei der Verstärkungsbereich sich über die gesamte in der Längsrichtung (X1) gemessene Länge (L) des Stoffs und über eine Breite (l1, l2) erstreckt, die strikt kleiner ist als die in der seitlichen Richtung (Y1) gemessene Breite (l) des Stoffs, und
- mindestens einen nicht verstärkten Bereich (40),
wobei die Dehnung des verstärkten Bereichs (20a, 20b, 20c, 20d) unter der Wirkung einer gegebenen Kraft, die in der Längsrichtung (X1) ausgeübt wird, kleiner ist als die Dehnung des nicht verstärkten Bereichs (40) unter der Wirkung der gleichen Kraft,
die geometrischen Formen (24) des Verstärkungsmotivs (22) diskrete Elemente sind und
die geometrischen Formen (24) des Verstärkungsmotivs (22) von jedem Verstärkungsbereich (20a, 20b, 20c, 20d) derart angeordnet sind, dass jede Gerade (D1), die sich in der seitlichen Richtung (Y1) des Stoffs erstreckt, mindestens eine von den Formen (24) schneidet.

2. Stoff (10, 110, 110', 210, 210') nach Anspruch 1, wobei der Bindungsgrad des Verstärkungsbereichs (20a, 20b, 20c, 20d) größer als 10%, bevorzugter größer als 15% ist.

3. Stoff (10, 110, 110', 210, 210') nach Anspruch 1 oder 2, wobei der Bindungsgrad in einem Nutzabschnitt des Verstärkungsbereichs (20a, 20b, 20c, 20d) größer als 15%, bevorzugter größer als 20% ist und/oder wobei der Bindungsgrad in einem Nutzabschnitt des Verstärkungsbereichs kleiner als 90% ist.

4. Stoff (10, 110, 110', 210, 210') nach einem der Ansprüche 1 bis 3, wobei die Breite (l1, l2) von jedem Verstärkungsbereich (20a, 20b, 20c, 20d) höchstens 80% der Breite (I) des Stoffs, vorzugsweise höchstens 60% der Breite (I) des Stoffs darstellt.

5. Stoff (10, 110, 110', 210, 210') nach einem der Ansprüche 1 bis 4, wobei die Dehnung des Verstärkungsbereichs (20a, 20b, 20c, 20d) bei 5N kleiner als die Dehnung des nicht verstärkten Bereichs (40) bei 5N, vorzugsweise kleiner als mindestens 5% der Dehnung des nicht verstärkten Bereichs (40) ist.

6. Stoff (10, 110, 110', 210, 210') nach einem der Ansprüche 1 bis 5, der aus einem Vlies vom kardierten, verfestigten Typ, insbesondere einem Vlies vom Typ Spunlace, besteht.

7. Laminierte Einheit (100, 200), die mindestens einen ersten Stoff (110, 210) nach einem der vorhergehenden Ansprüche und mindestens einen mit dem ersten Stoff verbundenen elastischen Film (150, 152, 250, 252) umfasst.

8. Laminierte Einheit (100, 200) nach Anspruch 7, die mindestens einen zweiten Vliesstoff (110', 210') umfasst, wobei der mindestens eine elastische Film (150, 152, 250, 252) zwischen dem ersten und dem zweiten Stoff (110, 110', 210, 210') angeordnet ist.

9. Laminierte Einheit (100, 200) nach Anspruch 8, wobei der erste Stoff (110, 210) mindestens einen ersten Verstärkungsbereich (120a, 120b, 220a, 220b) umfasst und der zweite Stoff (110', 210') ein Stoff nach einem der Ansprüche 1 bis 9 ist, der mindestens einen zweiten Verstärkungsbereich (120', 220a', 220b') umfasst.

10. Laminierte Einheit (100, 200) nach Anspruch 9, wobei der erste und der zweite Verstärkungsbereich (120a, 120', 220a, 220a') sich auf mindestens einem Überlappungsbereich (ZC) mit einer vorbestimmten Breite (lc) überlappen.

11. Laminierte Einheit (200) nach Anspruch 10, wobei die Projektionen der jeweiligen Verstärkungsmotive (222A, 222A') des ersten und des zweiten Verstärkungsbereichs (220a, 220a') auf mindestens einem Bereich der laminierten Einheit zusammenfallen.

12. Laminierte Einheit (100, 200) nach Anspruch 10, wobei die Projektionen der jeweiligen Verstärkungsmotive (122A, 122B) des ersten und des zweiten Verstärkungsbereichs in der Richtung Z derart angeordnet sind, dass jede Gerade, die sich in der seitlichen Richtung des Stoffs erstreckt, die Projektion der geometrischen Formen der Verstärkungsmotive des ersten und des zweiten Verstärkungsbereichs schneidet.

13. Laminierte Einheit (100, 200) nach einem der Ansprüche 7 bis 12, wobei jeder Verstärkungsbereich (120a, 120b, 220a, 220b; 120', 220a', 220b') von jedem Stoff (110, 110', 210, 210') in der seitlichen Richtung (Y1) in Bezug zu einem elastischen Abschnitt (E) der laminierten Einheit (100, 200) versetzt ist.

14. Verfahren zur Behandlung eines Vliesstoffs (10), wobei der Stoff sich entlang einer Längsrichtung (X1) und einer seitlichen Richtung (Y1) erstreckt, die orthogonal zur Längsrichtung (X1) ist, wobei das Verfahren mindestens einen Verstärkungsschritt umfasst, während dem auf mindestens einem Verstärkungsbereich (20) des Stoffs (10), der sich über die gesamte in der Längsrichtung gemessene Länge des Stoffs und über eine Breite (I1) erstreckt, die strikt kleiner als die Breite (I) des Stoffs (10), gemessen in seiner seitlichen Richtung, ist, die Fasern und/oder Filamente, die den Stoff (10) bilden, gemäß einem Verstärkungsmotiv (22) verbunden werden, das mehrere geometrische Formen (24) umfasst, wobei die geometrischen Formen (24) des Verstärkungsmotivs (22) diskrete Elemente sind und die geometrischen Formen (24) des Verstärkungsmotivs (22) von jedem Verstärkungsbereich (20a, 20b, 20c, 20d) derart angeordnet sind, dass jede Gerade (D1), die sich in der seitlichen Richtung (Y1) des Stoffs erstreckt, mindestens eine von den Formen (24) schneidet, derart dass die Dehnung des verstärkten Bereichs unter der Wirkung einer gegebenen, in der Längsrichtung ausgeübten Kraft kleiner ist als die Dehnung eines nicht verstärkten Bereichs des Stoffs unter der Wirkung der gleichen Kraft, wobei der Verstärkungsschritt vorzugsweise durch Heißkalandrierung ausgeführt wird.

15. Behandlungsverfahren nach Anspruch 14, das ferner vor dem Verstärkungsschritt einen Schritt der Anpassung der Breite (I) des Stoffs umfasst.

## Claims

1. A non-woven sheet (10, 110, 110', 210, 210') extending in a longitudinal direction (X1) and a lateral direction (Y1) orthogonal to the longitudinal direction (X1), said sheet comprising:
- at least one zone of reinforcement (20a, 20b, 20c) in which fibers and/or filaments constituting the sheet are bonded together in a reinforcing pattern (22) comprising a plurality of geometric shapes (24), the zone of reinforcement extending over the entire length (L) of the sheet measured in the longitudinal direction (X1), and over a width (ℓ1, ℓ2) strictly less than the width (ℓ) of the sheet measured in the lateral direction (Y1); and
- at least one non-reinforced zone (40);
the elongation of the reinforced zone (20a, 20b, 20c, 20d) under the effect of a given force exerted in the longitudinal direction (X1) being less than the elongation of the non-reinforced zone (40) under the effect of the same force,
the geometric shapes (24) of the reinforcing pattern (22) being discrete elements, and
the geometric shapes (24) of the reinforcing pattern (22) in each zone of reinforcement (20a, 20b, 20c, 20d) being arranged in such a manner that any straight line (D1) extending in the lateral direction (Y1) of the sheet intersects at least one of said shapes (24).

2. A sheet (10, 110, 110', 210, 210') according to claim 1, wherein the bonding percentage in the zone of reinforcement (20a, 20b, 20c, 20d) is greater than 10%, more preferably greater than 15%.

3. A sheet (10, 110, 110', 210, 210') according to claim 1 or 2, wherein the bonding percentage in a useful portion of the zone of reinforcement (20a, 20b, 20c, 20d) is greater than 15%, more preferably greater than 20%.

4. A sheet (10, 110, 110', 210, 210') according to any one of claims 1 to 3, wherein the width (ℓ1, ℓ2) of each zone of reinforcement (20a, 20b, 20c, 20d) represents at most 80% of the width (ℓ) of the sheet, preferably at most 60% of the width (ℓ) of the sheet.

5. A sheet (10, 110, 110', 210, 210') according to any one of claims 1 to 4, wherein the elongation at 5 N of the zone of reinforcement (20a, 20b, 20c, 20d) is less than the elongation at 5 N of the non-reinforced zone (40), preferably at least 5% less than the elongation of the non-reinforced zone (40).

6. A sheet (10, 110, 110', 210, 210') according to any one of claims 1 to 5, consisting in a consolidated carded type non-woven sheet, in particular a non-woven sheet of the Spunlace type.

7. A laminated assembly (100, 200) comprising at least a first sheet (110, 210) according to any preceding claim, and at least one elastic film (150, 152, 250, 252) connected to said first sheet.

8. A laminated assembly (100, 200) according to claim 7, comprising at least a second non-woven sheet (110', 210'), the at least one elastic film (150, 152, 250, 252) being interposed between the first and second sheets (110, 110', 210, 210').

9. A laminated assembly (100, 200) according to claim 8, wherein the first sheet (110, 210) comprises at least one first zone of reinforcement (120a, 120b, 220a, 220b), and the second sheet (110', 210') is a sheet according to any one of claims 1 to 9 having at least one second zone of reinforcement (120', 220a', 220b').

10. A laminated assembly (100, 200) according to claim 9, wherein the first and second zones of reinforcement (120a, 120', 220a, 220a') overlap over at least one overlap zone (ZC) of predetermined width (ℓc).

11. A laminated assembly (200) according to claim 10, wherein the projections of the respective reinforcing patterns (222A, 222A') of the first and second zones of reinforcement (220a, 220a') coincide over at least one zone of the laminated assembly.

12. A laminated assembly (100, 200) according to claim 10, wherein the projection of the respective reinforcing patterns (122A, 122B) of the first and second zones of reinforcement in the Z direction are arranged in such a manner that any straight line extending in the lateral direction of the sheet intersects the projections of the geometric shapes of the reinforcing patterns of the first and second zones of reinforcement.

13. A laminated assembly (100, 200) according to any one of claims 7 to 12, wherein each zone of reinforcement (120a, 120b, 220a, 220b; 120', 220a', 220b') of each sheet (110, 110', 210, 210') is offset in the lateral direction (Y1) relative to an elastic portion (E) of the laminated assembly (100, 200).

14. A method of processing a non-woven sheet (10), said sheet extending in a longitudinal direction (X1) and in a lateral direction (Y1) orthogonal to the longitudinal direction (X1), the method comprising at least one reinforcing step during which, over at least one zone of reinforcement (20) of the sheet (10) extending over the entire length of the sheet as measured in the longitudinal direction and over a width (ℓ1) that is strictly less than the width (ℓ) of the sheet (10) as measured in its lateral direction, fibers and/or filaments constituting the sheet (10) are bonded together using a reinforcing pattern (22) comprising a plurality of geometric shapes (24), the geometric shapes (24) of the reinforcing pattern (22) being discrete elements, and the geometric shapes (24) of the reinforcing pattern (22) in each zone of reinforcement (20a, 20b, 20c, 20d) being arranged in such a manner that any straight line (D1) extending in the lateral direction (Y1) of the sheet intersects at least one of said shapes (24), in such a manner that the elongation of the reinforced zone under the effect of a given force exerted in the longitudinal direction is less than the elongation of a non-reinforced zone of the sheet under the effect of the same force, the reinforcement step being preferably performed by hot calendering.

15. A processing method according to claim 14, further comprising, prior to the reinforcement step, a step of adjusting the width (ℓ) of the sheet.
